# EUROPEAN PATENT SPECIFICATION

(11) **EP 1 140 662 B1**
(45) Date of publication and mention of the grant of the patent: **03.05.2006**
(21) Application number: 99967289.2
(22) Date of filing: 14.12.1999
(51) Int. Cl.: B65D 85/16

(54) **PACKAGING OF FLEXIBLE ARTICLES**
VERPACKUNG VON FLEXIBLEN GEGENSTÄNDEN
EMBALLAGE D'ARTICLES SOUPLES

(30) Priority: 15.12.1998 US 112273 P; 08.11.1999 US 435759
(43) Date of publication of application: 10.10.2001
(73) Proprietor: KIMBERLY-CLARK WORLDWIDE, INC., Neenah, Wisconsin 54956 (US)
(72) Inventor: TIPPEY, Darold, Dean, Appleton, WI 54915 (US); BORDAIN, Nefetari, Edris, Dunwoody, GA 30350 (US)
(74) Representative: Davies, Christopher Robert
(86) International application number: PCT/US1999/029522
(87) International publication number: WO 2000/035776

(56) References cited:
- EP-A- 0 391 460
- EP-A- 0 618 148
- EP-A- 0 780 325
- DE-A- 2 614 235

## Description

The invention relates to the packaging of flexible absorbent articles including diapers, underpants, undergarments, or briefs. The packaging comprises a flexible outer casing in which the absorbent articles are arranged within the outer casing such that the articles are placed in an array such that at least a portion of their front faces are in a contacting relationship.

In today's markets, product and material costs as well as handling, shipping and display concerns impact packaging users. Producers of consumer products, such as absorbent articles, desire to hold production's material cost to a minimum, such as balancing packaging appeal to customers with the lowest cost of packaging materials and production costs associated with each packaging design. In addition, handling, shipping, and display space constraints further the optimal use of packaging.

It is known that during packaging, variations in the arrangement of flexible articles, such as absorbent articles, within the packaging can result in situations in which the flexible outer casing is not filled to an optimal extent. In addition, if a high degree of compression occurs, certain regions of the articles may be affected, such as elastic elements, including waist bands, standup cuffs, and elasticized side panels, mechanical fasteners, and absorbent cores in absorbent products. This could cause damage to the articles or reduced performance of the articles.

When flexible disposable articles, including absorbent articles, are packaged, compressed or otherwise, the volume size differences or changes that may occur can lead both to "wedge-like" shaped packaging and to the articles popping out of the outer casing at an inopportune time or in an undesired manner, i.e. two or three at a time. To counteract such situations, special configurations of the articles within the outer casing can be taken to better distribute the mass or bulk of the absorbent articles. However, a typical consequence of such configurations may be the reduction of the speed of packaging, and accordingly, the speed of production would also be reduced. In addition, the difference in the caliper of the different parts of the articles, especially absorbent articles, may lead to relatively unstable and easily deformable packages. Therefore, the shipment, storage, and displaying of such unstable packages cause a variety of problems and difficulties. The problems discussed above can be exasterbated when compressed packaging is utilized.

It is known that an array of flexible articles, compressed or otherwise, comprising one or more unit packages that may be maintained in their packaged configuration by a paper or plastic wrapping. The whole array can be encircled in a flexible covering made from a film typically of a thermoplastic material. An alternative type of a package unit uses the configuration wherein the flexible articles are folded and packaged in a "head-to-tail" configuration. However such a configuration may require the presence of individual paper wrappers to maintain the array. A downside to such a configuration is that the consumer would have to tear open the outer plastic flexible covering and remove the inner paper or plastic wrappers placed across the width of the products. These concerns become even more important when compressed packaging is used.

EP 0780325 A1 discloses a package containing an array of absorbent articles.

In view of the above problems, the present invention seeks to provide a package comprising an array of flexible articles, compressed or otherwise, that:
A. makes efficient use of the available packaging volume by redistributing the orientation of the flexible articles before packaging;
B. can be, if desired, compressed to a relatively small volume without causing damage or a significant reduction in the performance of the absorbent articles;
C. improves the optimal use of the free space inside outer casing and shipping units (corrugated cases) and improves pallet usage;
D. Is stable and uniform with regards to shape (tending towards a rectangular, square, or other designated design for a better fit when the package is shipped, stored, or displayed) and provides for an improved appearance;
E. allows for easier consumer access to the articles, especially absorbent articles, contained within the flexible outer casing when the outer casing is opened;
F. allows ease in removal of a single absorbent article, for use from the package;
G. eliminates the need for inner shapes, wrappers, or compression as a means for maintaining a desired configuration of the articles;
H. reduces material and shipping costs in allowing more articles, especially absorbent articles, in smaller packages;
I. provides an easier process for placing the articles in the package; and,
J. distributes the compression force more uniformly across the package, eliminating distinct high and low compression areas within the package.

The invention further discloses a relatively simple and reliable method for the packaging (compressed or otherwise) of an array of flexible articles while maintaining or increasing the speed and efficiencies of production.

The present invention provides a package containing an array of flexible absorbent articles in accordance with claim 1, and a method for forming a package of flexible absorbent articles in accordance with claim 14.

The present invention is a package comprising an array of flexible articles, which are housed in a flexible outer casing. The array of absorbent articles, has two regions, namely a first region and a second region. Each article comprises a top face, a bottom face, a front face, a back face, and a pair of side faces that are distributed over the first and second region of the array of articles. For simplicity, the relevant parts of the top, front, back, and side faces of the article are referred to as the upper section and the relevant parts of the bottom, front, back, and side faces of the article are referred to as the lower section. The upper and lower sections may have mutually different caliper, wherein caliper is understood to mean thickness or bulk. This is especially obvious in absorbent articles.

In standard packaging processes, the articles, such as absorbent articles, are aligned in an array wherein the back face of one article is in a contacting relationship with the front face of the adjacent article, and the bottom face of the first article is adjacent the bottom face of the adjacent article. According to the configuration of articles within the array of present invention, at least a portion or a predetermined number of articles within the array are in a front face to front face contacting relationship with adjacent articles (or alternatively, in a back face to back face contacting relationship). In addition, at least a portion or a predetermined number of the articles within the array may be in a top face to bottom face relationship with adjacent articles. Typically, the articles in a front face to front face contacting relationship with adjacent articles (or alternatively, in a back face to back face contacting relationship) are the articles in the top face to bottom face relationship with the adjacent articles.

The difference in the size (or the compression force for compression of the absorbent articles, when compressed packaging is being utilized) of the first and second regions when the upper and lower sections are distributed throughout the array of articles is at least 10% smaller than the difference in the size of the articles (or the compression force for compression of the articles when compressed packaging is being utilized) of the largest region when all the upper sections of the articles are located in the same region of the array of the articles. When a compression force is applied to the articles, the articles are compressed to between about 10% to about 75% of their uncompressed volume.

By redistributing the orientation or configuration of the articles within the array before packaging, the packaging of the array of the articles becomes more uniform. In the situations when compressed packaging is being utilized, the array of articles is typically reoriented before the compression force is applied. While it is not necessary to reorient the articles within the array before the compression force is applied to the array, the array of articles will be more stable for processing if the array is reoriented prior to the compression. The difference in the size of the upper and lower sections of the articles in the reoriented array, in order to obtain an equal distribution of the articles or volume of the first and second regions, is therefore reduced.

Such a reorientation provides for an optimal use of volume or space within the outer casing. This reorientation also prevents over-compression of various parts of the articles where compressed packaging is being utilized. The reorientation reduces or prevents damage to the absorbent articles, and maintains or improves the performance of the absorbent articles. In addition, the tendency for the articles to pop out of the outer casing during handling is reduced.

Using the reoriented configuration of the present invention for the array of articles, more articles can be included in a single array before the array becomes unstable. In this way, the packaging process is simplified and the speed of the production of the articles is maintained or increased while providing a more stable product package having the advantages stated above.

In addition, an improvement in the load bearing properties and shape stability of the package occurs. The packages of the reoriented array of absorbent articles, can be stacked in a more stable manner for shipping, handling, and display. It has also been found that a package according to the present invention can be compressed by at least 10% more in the direction of compression in comparison to a packaging comprising an equal number of articles wherein all the upper sections are located in the same region of the array of the articles.

In one embodiment of packaging according to the present invention, the articles are distributed within the array in such a manner that the size (or the compression forces when compressed packaging is utilized) for the first and second regions of the array of the articles are substantially equal. In this way, it is possible to simplify the packaging apparatus to accommodate the need for a variety of bag sizes. In the case when compressed packaging is utilized, the compression apparatus can be simplified as the pivoting preventive support for the compression plates to accommodate the different compressibilities of the array of articles can be reduced.

Alternatively, the array of the absorbent articles, may be oriented in such a manner that after compression the expansion force of the first region of the array is substantially equal to the expansion force of the second region in order to counteract deformation of the package upon removal of the compression forces.

An array of articles can be formed by stacking bi-folded absorbent articles, such as diapers, together, which have either non-uniform caliper or have low and high density regions. As it is known that a bi-folded diaper is folded once on itself at its crotch region. Such bi-folded diapers typically have a rounded upper section having a high compression resistance, which corresponds to the crotch region of the unfolded diaper, and a lower section with a low compression resistance, which corresponds to the waist regions of the unfolded diaper. The maximum improvement in the packaging can be achieved when the orientation of the rounded upper sections is alternated within the array of the absorbent articles. The orientation however of the upper sections may also be alternated for groups of two or more articles, and the number of rounded upper sections in the first and second regions of the array of compressed diapers need not necessarily be equal. Absorbent articles, to be considered may also be tri-folded, bi-tri-folded, or be folded by any of a variety of folds.

The term "compressibility" is intended to mean the reduction in volume when a predetermined force is applied to an article or to an array of articles, especially absorbent articles. This reduction in volume may be between 20% and 70% of the uncompressed volume.

A better understanding of the present invention can be achieved in conjunction with the study of the attached drawings.
- **FIGURE 1**: shows a known package comprising an array of flexible articles, such absorbent articles;
- **FIGURE 2**: shows a perspective view of folded articles having upper and lower sections of similar compressibilities or caliper;
- **FIGURE 3**: shows a perspective view of folded articles, such as absorbent articles, having upper and lower sections of different compressibilities or caliper;
- **FIGURE 4**: shows a perspective view of folded articles, such as absorbent articles, having upper and lower sections of different compressibilities or caliper;
- **FIGURE 5**: shows two packages of articles, such as absorbent articles, having deformed corners due to a higher compressibility in the lower regions of the packages;
- **FIGURE 6**: shows a packaging apparatus;
- **FIGURE 7**: shows a compression packaging apparatus;
- **FIGURE 8**: shows a compression packaging apparatus;
- **FIGURE 9**: schematically shows arrays of articles, such as absorbent articles, having differently distributed orientations;
- **FIGURE 10**: schematically shows arrays of articles, such as absorbent articles, having differently distributed orientations;
- **FIGURE 11**: schematically shows arrays of articles, such as absorbent articles, having different distributed orientations;
- **FIGURE 11a**: schematically shows arrays of articles, such as absorbent articles, having differently distributed orientations;
- **FIGURE 11b**: schematically shows arrays of articles, such as absorbent articles, having differently distributed orientations;
- **FIGURE 12**: schematically shows arrays of articles, such as absorbent articles, having differently distributed orientations;
- **FIGURE 13**: shows a plan view of a disposable diaper in the flattened state; and
- **FIGURE 14**: shows a cross-sectional view of the diaper taken from the view line 13 - 13' in **FIGURE 13.**
It will be appreciated that the present invention relates to an array of flexible absorbent articles in the form of diapers, underpants, undergarments or briefs.

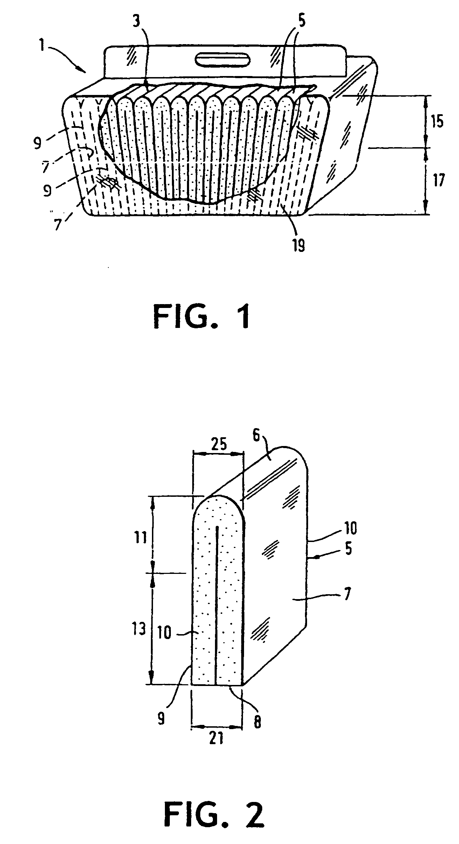

**FIGURE 1** shows a package **1** comprising an array **3** of folded, flexible articles **5,** such as absorbent articles, the array **3** shows a first region **15** and a second region **17.** The articles **5** may be compressed within the package **1.** In accordance with the invention, the articles **5** may comprise absorbent diapers, underpants, undergarments or briefs. When compressed, the articles **5,** especially absorbent articles, are compressed to between about 0% and about 90%, more typically about 0% and about 80%, more typically about 10% and 75%, and most typically about 20% and 70% of their uncompressed volume. The articles **5** are contained within a flexible outer casing **19,** with portions of the structure of the flexible outer casing **19** being cut away in **FIGURE 1** to more clearly show the array **3** of the articles **5,** in this case absorbent articles, within the package **1.** The flexible outer casing **19** maintains the array **3** of articles **5** (compressed or otherwise), and may comprise a thermoplastic bag or a paper bag, as it is known in the art.

In standard packaging processes, the articles **5** are aligned in an array **3** wherein the back face **9** of one article **5** is in a contacting relationship with the front face **7** of the adjacent article **5,** and the bottom face **8** of the first article **5** is adjacent the bottom face **8** of the adjacent article **5.**

According to the configuration of articles **5,** such as absorbent articles, within the array **3** of the package **1** of the present invention, at least a portion or a predetermined number of articles **5** within the array **3** are in a front face **7** to front face **7** contacting relationship with adjacent articles **5** (or alternatively, in a back face **9** to back face **9** contacting relationship). In addition, at least a portion or a predetermined number of the articles **5** within the array **3** may be in a top face **6** to bottom face **8** relationship with adjacent articles **5.** The two portions of articles **5** may or may not be the same articles **5.**

Typically, according to the present invention, the articles **5** in a front face **7** to front face **7** contacting relationship with adjacent articles **5** (or alternatively, in a back face **9** to back face **9** contacting relationship) are the articles **5** in the top face **6** to bottom face **8** relationship with the adjacent articles **5.** In such a configuration, the articles **5** in the front face **7** to front face **7** contacting relationship with adjacent articles **5** (or alternatively, in a back face **9** to back face **9** contacting relationship) would also be the articles **5** within the array **3** in a top face **6** to bottom face **8** relationship with adjacent articles **5.**

However, the articles **5** in the front face **7** to front face **7** contacting relationship with adjacent articles **5** (or alternatively, in a back face **9** to back face **9** contacting relationship) are not required to be the articles **5** within the array **3** are in a top face **6** to bottom face **8** relationship with adjacent articles **5.**

Between **10** and **100** absorbent articles **5** are typically comprised in the array **3,** As illustrated in **FIGURE 2,** each folded article **5** comprises a front face **7,** a back face **9,** a top face **6,** a bottom face **8,** and a pair of side faces **10.** Within the array **3,** the articles **5** are placed with at least a portion of their front faces **7** in a contacting relationship. Similarly, at least of a portion of the back faces **9** of the articles **5** are in contacting relationship. Each article **5** comprises an upper section **11** and a lower section **13**.

In the embodiment of **FIGURE 1**, all the upper sections **11** are located in the first region **15** of the array **3** of articles **5.** As the first region **15** of the array **3** of the articles **5** has a larger size (or a higher compression resistance when considering compressed packaging) than the second region **17,** the first region **15** of the *array* **3** of the articles **5** will have a larger size or volume than the second region **17**. In compressed packaging, the first region **15** of the array **3** of the articles **5**, especially when the articles **5** are absorbent, will have a larger size or volume after compression than the second region **17**. This may result in a package **1** of irregular or non-uniform dimensions (non-rectangular, non-square, ect.). Under such situations, the second region **17** of the package **1** will not be filled to an optimal extent. For example, when a rectangular shaped outer casing **19** is used for the package **1**, the second region **17** of the package **1** will not be filled to an optimal extent.

**FIGURE 2** shows a folded flexible article **5**, such as an absorbent article, which is of uniform caliper, i.e., the caliper **25** of the upper section **11** is substantially equal to the caliper **21** of the lower section **13**. When different materials are incorporated into the upper section **11** than in the lower section **13**, mutually different caliper may result. For example, the upper section **11** may comprise a higher concentration of absorbent gelling material particles or may comprise a resilient liquid acquisition material such as a foamed or a crosslinked cellulose material. Alternatively, the method or configuration used in folding the article **5**, may result in the upper section **11** and the lower section **13** having mutually different caliper. Thus, after folding the articles **5**, the caliper **25** for the upper section **11** and the caliper **21** for the lower section **13** of each article **5** and the dimensions of the first region **15** and the second region **17** of the array **3** of the articles **5**, along a direction which is orthogonal to the front faces **7** and the back faces **9** of the articles **5**, will be different.

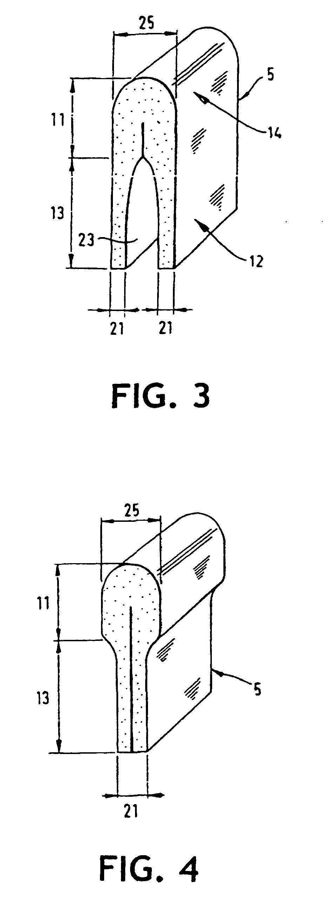

**FIGURE 3** shows another embodiment of a folded article **5** wherein the lower section **13** comprises a gap **23** such that the size of the lower section **13** is decreased in comparison to the size of the upper section **11**. Alternatively, if compressed packaging is being utilized, the embodiment of the folded article **5** wherein the lower section **13** comprises a gap **23** such that the compressibility of the lower section **13** is increased in comparison to the compressibility of the upper section **11**. For many bi-folded articles **5**, especially absorbent articles, the configuration of **FIGURE 3** will result as the caliper **25** corresponding to the crotch regions **14** of an article **5** is generally greater than the caliper **21** corresponding to the waist regions **12** of the articles **5**. In the folded article **5** of **FIGURE 3**, the crotch region **14** forms the upper section **11** and the waist regions **12** form the lower section **13**.

In **FIGURE 4,** a folded article **5** is shown which has a smaller caliper **21** for the lower section **13** in comparison to the caliper **25** of the upper section **11**. When the absorbent articles **5** of **FIGURES 2, 3**, and **4** are stacked in an array **3** of the articles **5,** as shown in **FIGURE 1,** the sizes will be different for the first and second regions **15** and **17** of the array **3** of the absorbent articles **5.** When the articles **5** are stacked as such and subsequently compressed, the compression force will be different for the first and second regions **15** and **17** of the array **3** of the articles **5**. These situations typically result in a package **1** of irregular dimensions, especially where the articles **5** are absorbent. Alternatively, the package **1** may have uniform or regular (such as rectangular or square) dimensions that are easily deformable in the second region **17.**

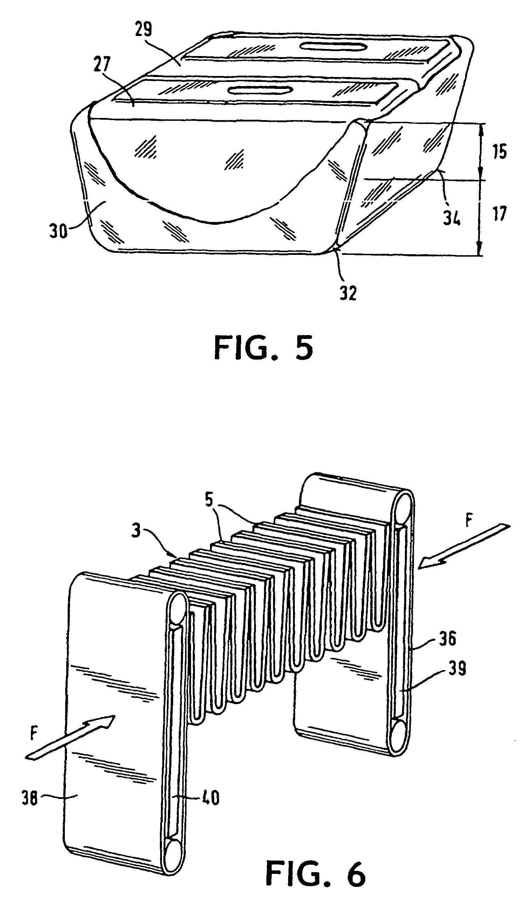

**FIGURE 5** shows a configuration wherein two packages **27** and **29**, each similar to package **1**, are combined by means of a stretch wrap film **30**. In situations where the size of the first region **15** of the packages **27** and **29** is greater than the size of the second region **17,** the packages **27** and **29** are deformed at their second regions **17.** Alternatively, when compressed packaging is being utilized and the compressibility of the first region **15** of the packages **27** and **29** is less than the compressibility of the second region **17**, the packages **27** and **29** are deformed at their second regions **17**. Where compressed packaging is used, the articles **5** within the packages **27** and **29,** will have an expansion force. The expansion force is a result of the compressed articles **5** returning or attempting to return to their original (non-compressed) state. The articles **5**, will therefore, fill the space provided within the package **27** or **29**. Rounded corners **32** and **34** are formed as the stretch wrap film **30** compresses the lower sections **13** of the articles **5** located in the second region **17**. Therefore, the combined packages **27** and **29** cannot be stacked with other similar packages in a stable manner.

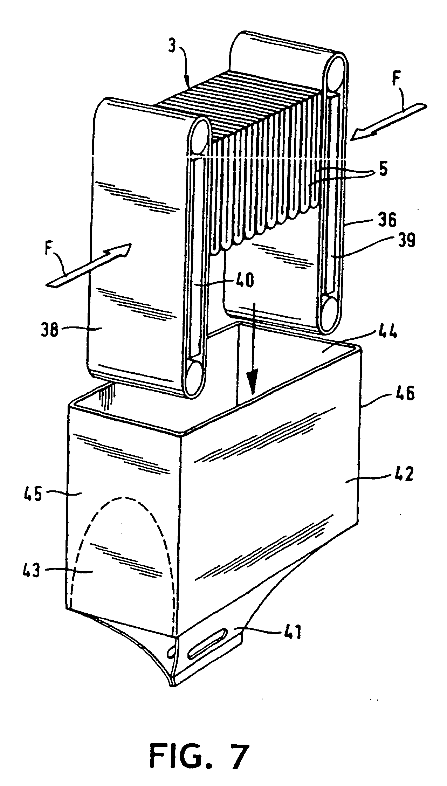

**FIGURE 6** shows a schematical view of how an array **3** of articles **5** is aligned between two belts **36** and **38**. Where compressed packaging is used, compression may be applied to individual articles **5**, articles **5** in an array **3**, or to both the individual article **5** and the array **3**. Where compression is applied to articles in an array, the belts **36** and **38** would be compression belts. Each belt **36** and **38** comprises a member **39** and **40**, respectively. Each belt **36** and **38** can provide compression force to the array **3** of the articles **5.** When compression force is applied, the array **3** of the articles **5** is compressed to between about 0% and 90%, more typically about 0% and about 80%, more typcially about 10% and 75%, most typically about 20% and about 70% of its uncompressed volume in the direction of the arrows F by moving the members **39** and **40** together with a force that can be as great as 2000 kg. After alignment or compression of the array **3**, the belts **36** and **38** are inserted through a bottom surface **44** of a bag **42**, as shown in **FIGURE 7.** The array **3** of articles **5** is inserted into the bag **42** by rotation of the belts **36** and **38**. After the array **3** has been inserted into the bag **42**, the belts **36** and **38** are retracted from the bag **42**, which is subsequently sealed on the bottom surface **44**. (Loading of an array **3** of the articles **5** can also be accomplished by using a pusher system.) The bag **42** comprises a handle **41** and an opening device **43**, which is formed by a line of weaknesses (perforations) on one of the side surfaces **45** or **46** of the bag **42**. This is one method of packaging compressed articles **5**. The invention is not intended to be limited to this particular embodiment for packaging compressed articles **5**.

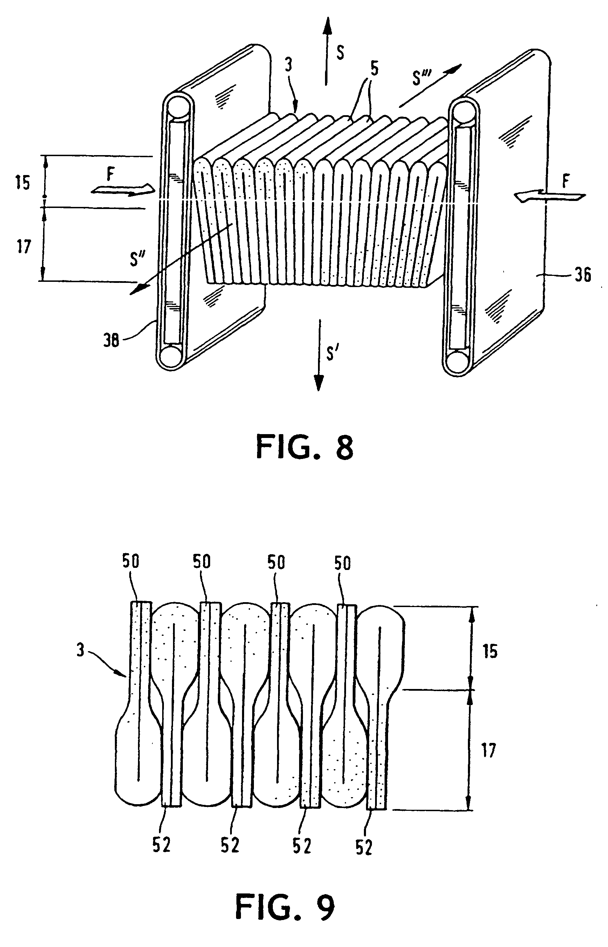

**FIGURE 8** shows that during compression of the array **3** of the articles **5** between the compression belts **36** and **38,** each article **5,** absorbent or otherwise, is squeezed tightly in the first region **15** of the array **3.** This will cause the articles **5** to be squeezed out of the array **3** in the direction of the arrows S or S', S" or S'" depending on the uniformity of the articles **5.** Due to the movement of the articles **5** within the array **3,** additional compression restraining means are necessary to prevent such a break up of the array **3**.

**FIGURE 9** shows the preferred orientation of the articles **50** and **52** according to the invention. The number of upper sections **11** and lower sections **13** of the articles **50** and **52** in the first region **15** of the array **3** is either equal to the number of upper sections **11** and lower sections **13** of the articles **50** and **52** in the second region **17** of the array **3** or it differs by one. In this way, the compression force necessary to compress the first region **15** is substantially equal to the compression force that is required to compress the second region **17** of the array **3**.

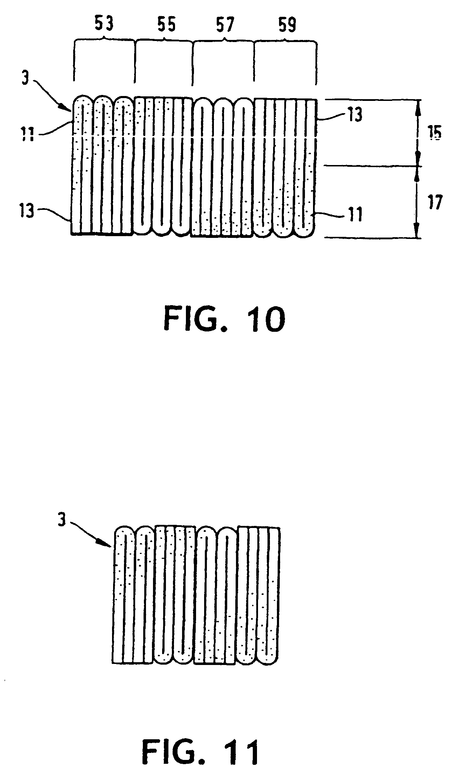

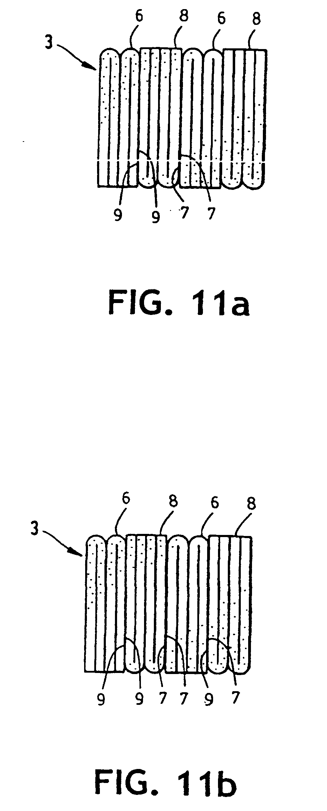

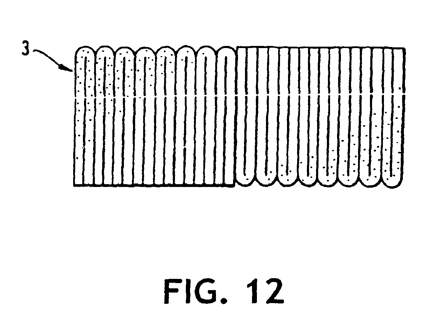

**In FIGURE 10,** the articles **5** are arranged into groups **53, 55, 57**, and **59** such that the upper sections **11** of the articles **5** in groups **53** and **57** are located in the first region **15** of the array **3** of the articles **5** and the upper sections **11** of the articles **5** in groups **55** and **59** are located in the second region **17** of the array **3** of the articles **5**. The number of the articles **5** comprised in each group may vary from **2**, as illustrated in **FIGURE 11**, to half the number of the articles **5** in the package **1**, as illustrated in **FIGURE 12**. The preferred embodiment occurs when the number of groups. are equal to each other, each group comprising a predetermined equal number of the articles **5**.

**FIGURES 11a** and **11b** show two of the possible different configurations of the articles **5** within the array **3** according to the invention. As discussed, shown in **FIGURE 11a**, the predetermined number of the articles **5** in the common orientation of the front face **7** to back face **9** contacting relationship of the articles **5** is changed to a front face **7** to front face **7** (or back face **9** to back face **9**) contacting relationship of the present invention may be the same predetermined number of the articles **5** wherein the bottom face **8** to bottom face **8** (or top face **6** to top face **6**) contacting relationship of the common orientation is changed to a bottom face **8** to top face **6** contacting relationship of the present invention. **FIGURE 11b** shows a configuration wherein the predetermined number of the articles **5** having a front face **7** to front face **7** (or back face **9** to back face **9**) contacting relationship may not be the same predetermined number of the articles **5** having the bottom face **8** to top face **6** contacting relationship of the present invention.

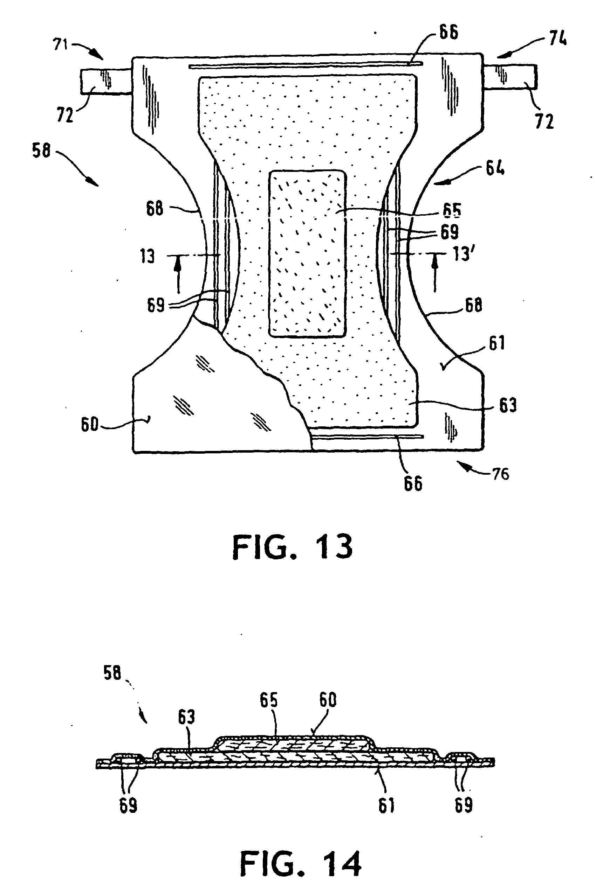

**FIGURE 13** shows a plan view of a flattened disposable article **58**, such as a diaper. The article **58** comprises a liquid pervious topsheet **60** and a liquid impervious backsheet **61**. In **FIGURE 13,** the topsheet **60** has been largely cut away to show the underlying features. The article **58** comprises an absorbent core **63**, which may comprise cellulosic fibres and hydrogel forming particles. (The absorbent core **63** may comprise any structure or material which is used for the absorption of bodily fluids. The discussion herein is not intended to limit the absorbent core **63** to the structure discussed.) A central acquisition patch **65** is placed in the crotch region **64** of the absorbent core **63**. Leg elastic elements **69** are located in the leg regions **68** of the article **58.** Front and back waist elastics **66** in the corresponding front and back regions **76** and **74** may be comprised in the article **58**. A fastening system **71**, which comprises adhesive tape fasteners **72**, is connected to the back region **74** of the article **58**. The fastening system **71** may alternatively comprise mechanical fasteners or a combination of adhesive fasteners **72** and mechanical fasteners.

**FIGURE 14** shows a cross-section of the article **58** taken from the view line 13-13' in **FIGURE 13**. On folding the article **58** along the line 13-13', the central region of the article **58,** comprising the acquisition patch **65,** will have the highest caliper.

The articles **5** discussed herein could be absorbent articles such as diapers, underpants, undergarments, or briefs. The articles **5** may be disposable, semi-durable, or durable in nature. It is anticipated that the present invention would be most beneficial wherein the articles **5** being packaged are non-uniform in shape once the articles **5** are folded.

The present invention is for a package **1** containing an array **3** of flexible absorbent articles **5** in the form of diapers, underpants, undergarments or briefs. The absorbent articles **5** comprise a front face **7,** a back face **9,** a top face **6,** a bottom face **8,** a pair of side faces **10,** an upper section **11,** and a lower section **13.** The sections **11** and **13** may have mutually different caliper **21** and **25**. According to the present invention, at least a portion of the absorbent articles **5** are placed with the front faces **7** in a contacting relationship and at least a portion of the back faces **9** are in a contacting relationship. The package **1** comprises a flexible outer casing **19**. The array **3** has a first region **15** and a second region **17**. The upper and lower sections **11** and **13** of the absorbent articles **5** are distributed over the first and second regions **11** and **13** of the array **3**. The distribution of upper and lower sections **11** and **13** is such that the difference in the size of the first and second regions **15** and **17** is at least about 10% smaller than when all of the upper sections **11** of the absorbent articles **5** are located in the same region **15** or **17** of the array **3**.

The present invention may include a configuration wherein the upper and lower sections **11** and **13** of the absorbent articles **5** are distributed in such a way that the sizes of the first and second regions **15** and **17** of the array **3** are substantially equal. The package **1** of the present invention may have at least a portion of the absorbent articles **5** placed with the bottom faces **8** in a contacting relationship with the top faces **6** of the adjacent absorbent articles **5**. The orientation of the absorbent articles **5** may be periodically alternated. The dimensions of the first region **15** of the array **3** may be substantially equal to the dimensions of the second region **17** of the array **3.** The absorbent articles **5** may comprise different caliper **21** or **25** in the upper and lower sections **11** and **13**. The package **1** of the present invention may not comprise a inner means, particularly not a paper wrapping to maintain the array **3** of the absorbent articles **5.**

The present invention is also for a method of forming a package **1** comprising the following steps:
a. transporting the absorbent articles **5** in a consecutive manner to a folding unit;
b. folding the absorbent articles **5**;
c. changing the orientation of the absorbent articles **5** at regularly spaced intervals;
d. aligning a predetermined number of the absorbent articles **5** with the front faces **7** or back faces **9** in a contacting relationship to form an uncompressed array **3**, wherein a predetermined number of the absorbent articles **5** have their upper sections **11** located in a first region **15** of the array **3** and a second predetermined number of the absorbent articles **5** have their upper sections **11** located in a second region **17** of the array **3**; and
e. placing the array **3** in a flexible outer casing **19**.

The present invention is also for a package **1** containing an array **3** of compressed, flexible absorbent articles **5** in the form of diapers, underpants, undergarments or briefs. The absorbent articles **5** comprise a front face **7**, a back face **9**, a top face **6**, a bottom face **8**, a pair of side faces **10**, an upper section **11**, and a lower section **13**. The sections **11** and **13** may have mutually different compressibilities and caliper **21** and **25**. According to the present invention, at least a portion of the absorbent articles **5** are placed with said front faces **7** in a contacting relationship and at least a portion of the back faces **9** in a contacting relationship. The package **1** comprises a flexible outer casing **19.** The array **3** has a first region **15** and a second region **17**. The upper and lower sections **11** and **13** of the absorbent articles **5** are distributed over the first and second regions **15** and **17** of the array **3**. The distribution of the upper and lower sections **11** and **13** is such that the difference in the compression force for compression of the first and second regions **15** and **17** to between 0% and 90% of their uncompressed volume is at least 10% smaller than the difference in the compression force for compression of said first and second regions **15** and **17** when all of the upper sections **11** of the absorbent articles **5** are located in the same region **15** or **17** of the array **3**. The flexible outer casing **19** maintains the array **3** of the compressed absorbent articles **5**.

The present invention may include a configuration wherein the upper and lower sections **11** and **13** of the absorbent articles **5** are distributed in such a way that the compression forces for said first and second regions **15** and **17** of the array **3** are substantially equal. The package **1** may have at least a portion of the absorbent articles **5** placed with the bottom faces **8** in a contacting relationship with the top faces **6** of the adjacent absorbent articles **5**. The orientation of the absorbent articles **5** may be periodically alternated. After compression, the dimension along the direction of compression of the first region **15** of the array **3** may be substantially equal to the dimension along the direction of compression of the second region **17** of the array **3**. After compression, the expansion force of the first region **15** may be substantially equal to the expansion force of the second region **17**. The articles **5** may comprise different caliper **21** and **25** in the upper and lower sections **11** and **13**. The package **1** may not comprise an inner means, particularly not a paper wrapping to maintain the array **3** of the compressed absorbent articles **5**.

The present invention is also for a method of forming a package **1** comprising the following steps:
a. transporting the absorbent articles **5** in a consecutive manner to a folding unit;
b. folding the absorbent articles **5**;
c. changing the orientation of the absorbent articles **5** at regularly spaced intervals;
d. aligning a predetermined number of the absorbent articles **5** with the front faces **7** or the back faces **9** in a contacting relationship to form an uncompressed array **3**, wherein a predetermined number of the absorbent articles **5** have their upper sections **11** located in a first region **15** of the array **3**;
e. compressing the array **3**; and
f. placing the compressed array **3** in a flexible outer casing **19.**

The method of forming a package **1** may also include a second predetermined number of the absorbent articles **5** having their upper sections **11** located in a second region **17** of the array **3**.

The method of forming a package **1** may also comprise the following steps:
a. transporting the absorbent articles **5** in a consecutive manner to a folding unit;
b. folding the absorbent articles **5**;
c. changing the orientation of the absorbent articles **5** at regularly spaced intervals;
d. aligning a predetermined number of the absorbent articles **5** with the front faces **7** or the back faces **9** in a contacting relationship to form an uncompressed array **3,** wherein a predetermined number of the absorbent articles **5** have their upper sections **11** located in a first region **15** of the array **3** and a second predetermined number of the absorbent articles **5** have their upper sections **11** located in a second region **17** of the array **3**;
e. compressing the array **3**; and,
f. placing the compressed array **3** in a flexible outer casing **19.**

The method of forming a package **1** wherein the compression force is substantially uniform across the first and second regions **15** and **17**.

While the invention has been described in detail with respect to specific embodiments thereof, it will be apparent to those skilled in the art that various alternations, modifications an other changes may be made to the invention without departing from the scope of the present invention. It is therefore intended that the claims cover all such modifications, alterations and other changes encompassed by the appended claims.

## Claims

1. A package (1) containing an array (3) of flexible absorbent articles (5), said absorbent articles being diapers, underpants, undergarments or briefs comprising a front face (7), a back face (9), a top face (6), a bottom face (8), side faces (10), an upper section (11) and a lower section (13), said sections having mutually different caliper, said package comprising a flexible outer casing (19), said array having a first region (15) and a second region (17) and said upper and lower sections of said articles being distributed over said first and second regions of said array (3),
said distribution of upper (11) and lower sections (13), being such that the difference in the size of said first and second regions is at least about 10% smaller than when all of said upper section (11) of said articles are located in the same region of said array,
**characterised in that** at least a portion of said articles are placed in said array with said front faces (7) in a contacting relationship and at least a portion of said back faces (9) in a contacting relationship.

2. A package according to Claim 1, wherein said upper (11) and lower sections (13) of said articles are distributed in such a way that the sizes of said first and second regions of said array are substantially equal.

3. A package according to Claim 1 or 2, wherein at least a portion of said articles are placed with said bottom faces (8) in a contacting relationship with said top faces (6) of adjacent articles.

4. The package according to Claim 3, wherein those articles (5) in a front face (7) to front face (7) or back face (9) to back face (9) contacting relationship are those articles (5) in top face (6) to bottom face (8) contacting relationship.

5. A package according to Claim 1, 2, 3 or 4, wherein the orientation of said articles is periodically alternated.

6. A package according to any preceding claim, wherein the dimensions of said first region (15) of said array is substantially equal to the dimensions of said second region (17) of said array.

7. A package according to any preceding claim, wherein said articles comprise different caliper in said upper (11) and lower (13) sections.

8. A package according to any preceding claim, wherein said package does not comprise an inner means, particularly not a paper wrapping to maintain said array of said absorbent articles.

9. A package in accordance with any preceding claim, wherein said array is an array of compressed, flexible articles.

10. The package of Claim 9, wherein said distribution of said upper and lower sections, is such that the difference in the compression force for compression of said first and second regions to between 0% and 90% of their uncompressed volume, is at least 10% smaller than the difference in the compression force of said first and second regions, when all of said upper sections of said articles are located in the same region of said array, and said flexible outer casing maintains an array of said compressed articles.

11. A package according to Claim 10, wherein said upper and lower sections of said articles are distributed in such a way that the compression forces for said first and second regions of said array are substantially equal.

12. A package according to Claim 10 or 11, wherein, after compression, the dimension along the direction of compression of said first region of said array is substantially equal to the dimension along the direction of compression of said second region of said array.

13. A package according to Claim 10, 11 or 12, wherein after compression the expansion force of said first region is substantially equal to the expansion force of said second region.

14. A method of forming a packing of flexible absorbent articles, said articles being diapers, underpants, undergarments or briefs comprising a front face (7), a back face (9), a top face (6), a bottom face (8), side faces (10) an upper section (11) and a lower section (13), said sections having mutually different caliper, the method comprising the following steps:
a. transporting said articles (5) in a consecutive manner to a folding unit;
b. folding said articles (5);
c. changing the orientation of said articles (5) at regularly spaced intervals;
d. aligning said articles to form an uncompressed array wherein a predetermined number of said articles have their said upper sections (11) located in a first region (15) of said array and a second predetermined number of said articles have their said upper sections (11) located in a second region (17) of said array; and
e. placing said array in a flexible outer casing (19);
**characterised in that** the method comprises the step of aligning said articles (5) in the array such that a predetermined number of said articles have said front faces or said back faces in a contacting relationship.

15. A method of forming a package in accordance with any of Claims 1 to 8, said method comprising the steps of Claim 14.

16. The method of forming a package according to Claim 14, further comprising compressing said array, and placing said compressed array in the flexible outer casing.

17. A method of forming a package according to any of Claims 9 to 13, said method comprising the steps of Claim 16.

18. A method according to Claim 16 or 17, wherein the compression force is substantially uniform across said first and second regions.

19. The package or method of any preceding claim, wherein said articles are disposable diapers, underpants, undergarments or briefs.

20. The package or method of any preceding claim, wherein said articles comprise a fastening system (71) connected to a back region (74) of the article.

21. The package or method of Claim 20, wherein said fastening system comprises fasteners which extend outwardly from the side edges of said back region (74) of the article (58).

22. The package or method of Claim 20 or Claim 21, wherein said fasteners are mechanical fasteners or adhesive tape fasteners.

23. The package or method of any of Claims 20 to 22, wherein the article (58) further comprises a liquid pervious topsheet (60), a liquid impervious backsheet (61) and an absorbent core (63), the article further comprising leg elastic elements (69) located in leg regions (68) of the article (58).

24. The package or method of Claim 23, further comprising a central acquisition patch (65) placed in the crotch region (64) of the absorbent core (63).

## Patentansprüche

1. Verpackung (1), die eine regelmäßige Anordnung (3) von flexiblen, saugfähigen Artikeln (5) beinhaltet, wobei die saugfähigen Artikel Windeln, Unterhosen, Unterbekleidung oder Slips sind, die eine Vorderseite (7), eine Rückseite (9), eine Oberseite (6), eine Unterseite (8), Seitenflächen (10), einen oberen Abschnitt (11) und einen unteren Abschnitt (13) umfassen, wobei die Abschnitte voneinander unterschiedliche Dickenmaße aufweisen, wobei die Verpackung eine flexible äußere Umhüllung (19) umfasst, wobei die regelmäßige Anordnung einen ersten Bereich (15) und einen zweiten Bereich (17) hat und wobei der obere und der untere Abschnitt der Artikel über den ersten und den zweiten Bereich der regelmäßigen Anordnung (3) verteilt sind,
wobei die Verteilung des oberen Abschnittes (11) und des unteren Abschnittes (13) derart ist, dass der Unterschied in der Größe des ersten und des zweiten Bereiches wenigstens etwa 10% kleiner ist, als wenn der gesamte obere Abschnitt (11) der Artikel in dem gleichen Bereich der regelmäßigen Anordnung angeordnet wäre,
**dadurch gekennzeichnet, dass** wenigstens ein Teil der Artikel in der regelmäßigen Anordnung mit den Vorderseiten (7) in einer berührenden Beziehung steht und dass wenigstens ein Teil der Rückseiten (9) in einer berührenden Beziehung steht.

2. Verpackung nach Anspruch 1, wobei die oberen Abschnitte (11) und die unteren Abschnitte (13) der Artikel so verteilt sind, dass die Größen des ersten Abschnittes und des zweiten Abschnittes der regelmäßigen Anordnung im Wesentlichen gleich sind.

3. Verpackung nach Anspruch 1 oder 2, wobei wenigstens ein Teil der Artikel mit den Unterseiten (8) in einer berührenden Beziehung mit den Oberseiten (6) von benachbarten Artikeln angeordnet ist.

4. Verpackung nach Anspruch 3, wobei diejenigen Artikel (5), die in einer berührenden Beziehung Vorderseite (7) zu Vorderseite (7) oder Rückseite (9) zu Rückseite (9) stehen, diejenigen Artikel sind, die in einer berührenden Beziehung Oberseite (6) zu Unterseite (8) stehen.

5. Verpackung nach Anspruch 1, 2, 3 oder 4, wobei die Ausrichtung der Artikel regelmäßig wiederkehrend gewechselt wird.

6. Verpackung nach einem der vorhergehenden Ansprüche, wobei die Abmessungen des ersten Bereiches (15) der regelmäßigen Anordnung im Wesentlichen gleich den Abmessungen des zweiten Bereiches (17) der regelmäßigen Anordnung sind.

7. Verpackung nach einem der vorhergehenden Ansprüche, wobei die Artikel verschiedene Dickenmaße in dem oberen Bereich (11) und in dem unteren Bereich (13) umfassen.

8. Verpackung nach einem der vorhergehenden Ansprüche, wobei die Verpackung keine inneren Mittel, insbesondere keine Papierverpackung, zur Aufrechterhaltung der regelmäßigen Anordnung der saugfähigen Artikel aufweist.

9. Verpackung nach einem der vorhergehenden Ansprüche, wobei die regelmäßige Anordnung eine Anordnung von zusammengedrückten, flexiblen Artikeln ist.

10. Verpackung aus Anspruch 9, wobei die Verteilung der oberen Abschnitte und der unteren Abschnitte derart ist, dass der Unterschied in der Druckkraft zum Zusammendrücken des ersten Bereiches und des zweiten Bereiches auf zwischen 0% und 90% ihres unverdichteten Volumens wenigstens 10% kleiner ist als der Unterschied in der Druckkraft des ersten Bereiches und des zweiten Bereiches, wenn alle oberen Abschnitte der Artikel in dem gleichen Bereich der regelmäßigen Anordnung angeordnet sind und wenn die flexible äußere Umhüllung eine regelmäßige Anordnung der zusammengedrückten Artikel aufrecht erhält.

11. Verpackung nach Anspruch 10, wobei die oberen Abschnitte und die unteren Abschnitte der Artikel so verteilt sind, dass die Druckkräfte für den ersten Bereich und für den zweiten Bereich der regelmäßigen Anordnung im Wesentlichen gleich sind.

12. Verpackung nach Anspruch 10 oder 11, wobei nach dem Zusammendrücken das Maß in der Richtung des Zusammendrückens des ersten Bereiches der regelmäßigen Anordnung im Wesentlichen gleich dem Maß in der Richtung des Zusammendrückens des zweiten Bereiches der regelmäßigen Anordnung ist.

13. Verpackung nach Anspruch 10, 11 oder 12, wobei nach dem Zusammendrücken die Dehnungskraft des ersten Bereiches im Wesentlichen gleich der Dehnungskraft des zweiten Bereiches ist.

14. Verfahren zum Ausbilden einer Verpackung für flexible, saugfähige Artikel, wobei die Artikel Windeln, Unterhosen, Unterbekleidung oder Slips sind, die eine Vorderseite (7), eine Rückseite (9), eine Oberseite (6), eine Unterseite (8), Seitenflächen (10), einen oberen Abschnitt (11) und einen unteren Abschnitt (13) umfassen, wobei die Abschnitte voneinander unterschiedliche Dickenmaße aufweisen, wobei das Verfahren die folgenden Schritte umfasst:
a. Transportieren der Artikel (5) in einer Aufeinanderfolge zu einer Zusammentegeeinheit;
b. Zusammenlegen der Artikel (5);
c. Ändern der Ausrichtung der Artikel (5) in regelmäßigen Abständen;
c. Ändem der Ausrichtung der Artikel (5) in regelmäßigen Abständen;
d. Ausrichten der Artikel, um eine unverdichtete regelmäßige Anordnung auszubilden, wobei die oberen Abschnitte (11) einer vorgegebenen Anzahl der Artikel in einem ersten Bereich (15) der regelmäßigen Anordnung angeordnet sind und wobei die oberen Abschnitte (11) einer vorgegebenen Anzahl der Artikel in einem zweiten Bereich (17) der regelmäßigen Anordnung angeordnet sind; und
e. Platzieren der regelmäßigen Anordnung in einer flexiblen äußeren Umhüllung (19);
**dadurch gekennzeichnet, dass** das Verfahren den Schritt des Ausrichtens der Artikel (5) in der regelmäßigen Anordnung in einer solchen Weise umfasst, dass die Vorderseiten oder die Rückseiten einer vorgegebenen Anzahl von Artikeln in berührender Beziehung vorliegen.

15. Verfahren des Ausbildens einer Verpackung nach einem der Ansprüche 1 bis 8, wobei das Verfahren die Schritte aus Anspruch 14 umfasst.

16. Verfahren des Ausbildens einer Verpackung nach Anspruch 14, das weiterhin das Zusammendrücken der regelmäßigen Anordnung und das Platzieren der verdichteten regelmäßigen Anordnung in der flexiblen äußeren Umhüllung umfasst.

17. Verfahren des Ausbildens einer Verpackung nach einem der Ansprüche 9 bis 13, wobei das Verfahren die Schritte aus Anspruch 16 umfasst.

18. Verfahren nach Anspruch 16 oder 17, wobei die Druckkraft über den ersten Bereich und den zweiten Bereich im Wesentlichen gleichmäßig ist.

19. Verpackung oder Verfahren aus einem der vorhergehenden Ansprüche, wobei die Artikel Wegwerfwindeln, Wegwerfunterhosen, Einwegunterkleidung oder Wegwerfslips sind.

20. Verpackung oder Verfahren nach einem der vorhergehenden Ansprüche, wobei die Artikel ein Befestigungssystem (71) umfassen, das mit einem Rückseitenbereich (74) des Artikels verbunden ist.

21. Verpackung oder Verfahren aus Anspruch 20, wobei das Befestigungssystem Befestigungsmittel umfasst, die sich von den Seitenrändem des Rückseitenbereiches (74) des Artikels (58) nach außen erstrecken.

22. Verpackung oder Verfahren aus Anspruch 20 oder Anspruch 21, wobei die Befestigungsmittel mechanische Befestigungsmittel oder Klebeband-Befestigungsmittel sind.

23. Verpackung oder Verfahren nach einem der Ansprüche 20 bis 22, wobei der Artikel (58) weiterhin eine flüssigkeitsdurchlässige obere Lage (60), eine flüssigkeitsdurchlässige rückseitige Lage (61) und einen saugfähigen Kern (63) umfasst, wobei der Artikel weiterhin elastische Beinelemente (69) umfasst, die sich in den Beinbereichen (68) des Artikels (58) befinden.

24. Verpackung oder Verfahren aus Anspruch 23, weiterhin umfassend eine zentrale Auffangfläche (65), die in dem Schrittbereich (64) des saugfähigen Kerns (63) angeordnet ist.

## Revendications

1. Emballage (1) contenant un réseau (3) d'articles absorbants souples (5), lesdits articles absorbants étant des langes, des slips, des sous-vêtements ou des caleçons présentant une face avant (7), une face arrière (9), une face supérieure (6), une face inférieure (8), des faces latérales (10), une section supérieure (11) et une section inférieure (13), lesdites sections présentant des épaisseurs mutuellement différentes, ledit emballage comprenant une enceinte extérieure souple (19), ledit réseau présentant une première région (15) et une deuxième région (17), et lesdites sections supérieure et inférieure desdits articles étant distribuées sur lesdites première et deuxième régions dudit réseau (3);
ladite distribution de sections supérieure (11) et inférieure (13) est telle que la différence de taille entre lesdites première et deuxième régions est au moins d'environ 10 % plus petite que lorsque la totalité de ladite section supérieure (11) desdits articles est située dans la même région dudit réseau;
**caractérisé en ce qu'**au moins une partie desdits articles sont placés dans ledit réseau avec lesdites faces avant (7) se trouvant dans une relation de contact et au moins une partie desdites faces arrière (9) se trouvant dans une relation de contact.

2. Emballage selon la revendication 1, dans lequel lesdites sections supérieure (11) et inférieure (13) desdits articles sont distribuées d'une manière telle que la taille desdites première et deuxième régions dudit réseau soit sensiblement égale.

3. Emballage selon la revendication 1 ou 2, dans lequel au moins une partie desdits articles sont disposés avec lesdites faces inférieures (8) dans une relation de contact avec lesdites faces supérieures (6) d'articles voisins.

4. Emballage selon la revendication 3, dans lequel les articles (5) se trouvant dans une relation de contact de face avant (7) à face avant (7) ou de face arrière (9) à face arrière (9) sont les articles (5) se trouvant dans une relation de contact de face supérieure (6) à face inférieure (8).

5. Emballage selon la revendication 1, 2, 3 ou 4, dans lequel l'orientation desdits articles est périodiquement alternée.

6. Emballage selon l'une quelconque des revendications précédentes, dans lequel les dimensions de ladite première région (15) dudit réseau sont sensiblement égales aux dimensions de ladite deuxième région (17) dudit réseau.

7. Emballage selon l'une quelconque des revendications précédentes, dans lequel lesdits articles présentent une épaisseur différente entre lesdites sections supérieure (11) et inférieure (13).

8. Emballage selon l'une quelconque des revendications précédentes, dans lequel ledit emballage ne comprend pas de moyens intérieurs, en particulier pas d'emballage en papier servant à maintenir ledit réseau d'articles absorbants.

9. Emballage selon l'une quelconque des revendications précédentes, dans lequel ledit réseau est un réseau d'articles souples comprimés.

10. Emballage selon la revendication 9, dans lequel ladite distribution desdits sections supérieure et inférieure est telle que la différence dans la force de compression pour la compression desdites première et deuxième régions jusque entre 0 % et 90 % de leur volume non comprimé, est au moins 10 % plus petite que la différence dans la force de compression desdites première et deuxième régions, lorsque la totalité desdites régions supérieures desdits articles sont situées dans la même région dudit réseau, et ladite enceinte extérieure souple maintient un réseau desdits articles comprimés.

11. Emballage selon la revendication 10, dans lequel lesdites sections supérieure et inférieure desdits articles sont distribuées d'une manière telle que les forces de compression pour lesdites première et deuxième régions dudit réseau soient sensiblement égales.

12. Emballage selon la revendication 10 ou 11, dans lequel, après la compression, la dimension le long de la direction de compression de ladite première région dudit réseau est sensiblement égale à la dimension le long de la direction de compression de ladite deuxième région dudit réseau.

13. Emballage selon la revendication 10, 11 ou 12, dans lequel, après la compression, la force d'expansion de ladite première région est sensiblement égale à la force d'expansion de ladite deuxième région.

14. Procédé pour former un emballage d'articles absorbants souples, lesdits articles étant des langes, des slips, des sous-vêtements ou des caleçons présentant une face avant (7), une face arrière (9), une face supérieure (6), une face inférieure (8), des faces latérales (10), une section supérieure (11) et une section inférieure (13), lesdites sections présentant des épaisseurs mutuellement différentes, le procédé comprenant les étapes suivantes:
a. transporter lesdits articles (5) de manière consécutive jusqu'à une unité de pliage;
b. plier lesdits articles (5);
c. changer l'orientation desdits articles (5) à des intervalles régulièrement espacés;
d. aligner lesdits articles pour former un réseau non comprimé dans lequel un nombre prédéterminé desdits articles ont leurs dites sections supérieures (11) situées dans une première région (15) dudit réseau, et un deuxième nombre prédéterminé desdits articles ont leurs dites sections supérieures (11) situées dans une deuxième région (17) dudit réseau; et
e. placer ledit réseau dans une enceinte extérieure souple (19);
**caractérisé en ce que** le procédé comprend l'étape d'alignement desdits articles (5) dans le réseau de telle sorte qu'un nombre prédéterminé desdits articles présentent lesdites faces avant ou lesdites faces arrière en relation de contact.

15. Procédé pour former un emballage selon l'une quelconque des revendications 1 à 8, ledit procédé comprenant les étapes décrites à la revendication 14.

16. Procédé pour former un emballage selon la revendication 14, comprenant en outre la compression dudit réseau, et le placement dudit réseau comprimé dans l'enceinte extérieure souple.

17. Procédé pour former un emballage selon l'une quelconque des revendications 9 à 13, ledit procédé comprenant les étapes décrites à la revendication 16.

18. Procédé pour former un emballage selon la revendication 16 ou 17, dans lequel la force de compression est sensiblement uniforme à travers lesdites première et deuxième régions.

19. Emballage ou procédé selon l'une quelconque des revendications précédentes, dans lequel lesdits articles sont des langes jetables, des slips, des sous-vêtements ou des caleçons.

20. Emballage ou procédé selon l'une quelconque des revendications précédentes, dans lequel lesdits articles comprennent un système de fixation (71) connecté à une région arrière (74) de l'article.

21. Emballage ou procédé selon la revendication 20, dans lequel ledit système de fixation comprend des attaches s'étendant vers l'extérieur à partir des bords latéraux de ladite région arrière (74) de l'article (58).

22. Emballage ou procédé selon la revendication 20 ou la revendication 21, dans lequel lesdites attaches sont des attaches mécaniques ou des attaches à ruban adhésif.

23. Emballage ou procédé selon l'une quelconque des revendications 20 à 22, dans lequel l'article (58) comprend en outre une feuille supérieure perméable aux liquides (60), une feuille arrière imperméable aux liquides (61) et un noyau absorbant (63), l'article comprenant en outre des éléments élastiques pour les jambes (69) situés dans la région des jambes (68) de l'article (58).

24. Emballage ou procédé selon la revendication 23, comprenant en outre un renfort de rétention central (65) placé dans la région d'entrejambe (64) du noyau absorbant (63).
